(19) **Europäisches Patentamt / European Patent Office / Office européen des brevets**

(11) **EP 3 090 261 B1**

(12) **EUROPEAN PATENT SPECIFICATION**

(45) Date of publication and mention
of the grant of the patent:
**20.03.2019 Bulletin 2019/12**

(51) Int Cl.:
**G01N 33/50** *(2006.01)*     **G01N 21/00** *(2006.01)*
**G01N 33/68** *(2006.01)*

(21) Application number: **15700814.5**

(22) Date of filing: **02.01.2015**

(86) International application number:
**PCT/US2015/010066**

(87) International publication number:
**WO 2015/103495 (09.07.2015 Gazette 2015/27)**

(54) **CONVERGENCE OF AGGREGATION RATE WITH VALIDATED PERIPHERAL DIAGNOSTICS FOR ALZHEIMER'S DISEASE**

KONVERGENZ VON AGGREGATIONSGESCHWINDIGKEIT MIT VALIDIERTEN PERIPHEREN DIAGNOSTISCHEN VERFAHREN FÜR ALZHEIMER

CONVERGENCE DU TAUX D'AGRÉGATION ASSOCIÉS À DES DIAGNOSTICS PÉRIPHÉRIQUES VALIDÉS DE LA MALADIE D'ALZHEIMER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.01.2014 US 201461923373 P**

(43) Date of publication of application:
**09.11.2016 Bulletin 2016/45**

(73) Proprietor: **Blanchette Rockefeller Neurosciences Institute**
**Morgantown, WV 26505-3409 (US)**

(72) Inventors:
• **CHIRILA, Florin V.**
**Morgantown, WV 26505 (US)**
• **KHAN, Tapan K.**
**Morgantown, WV 26505 (US)**

• **ALKON, Daniel**
**Chevy Chase, MD 20815 (US)**

(74) Representative: **Finnegan Europe LLP**
**1 London Bridge**
**London SE1 9BG (GB)**

(56) References cited:
**WO-A1-2011/041761     WO-A1-2012/155051**
**US-A1- 2007 082 366**

• **CHIRILA FLORIN V ET AL: "Spatiotemporal Complexity of Fibroblast Networks Screens for Alzheimer's Disease", JOURNAL OF ALZHEIMER'S DISEASE, IOS PRESS, NL, vol. 33, no. 1, 1 January 2013 (2013-01-01), pages 165-176, XP009182993, ISSN: 1387-2877**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

This application claims priority to U.S. Provisional Patent Application No. 61/923,373, filed January 3, 2014.

Alzheimer's disease (AD) is a neurodegenerative disorder characterized by the progressive decline of memory and cognitive functions. It is estimated that over five million Americans are living with this progressive and fatal disease. Alzheimer's destroys brain cells, causing memory loss and problems with thinking and behavior that decrease quality of life. AD has no known cure, but treatments for symptoms can improve the quality of life of the millions of people, and their families, suffering from AD. An early diagnosis of AD gives the patient time to make choices that maximize quality of life, reduces anxiety about unknown problems, gives more time to plan for the future, and provides a better chance of benefiting from treatment.

The inaccuracy of the diagnosis for AD, however, has made its therapeutic intervention difficult, particularly at early stages to prevent significant neurodegeneration and cognitive dysfunction. Thus, there is a need for highly sensitive and specific tests to diagnose Alzheimer's disease.

WO 2012/155051 reports a peripheral diagnostic method for screening Alzheimer's disease in patients based on quantitatively measured complexity of skin-sampled fibroblast networks.

In earlier studies of human dermal fibroblast aggregation, the present inventors quantified cell aggregation by the area of the unit aggregate, which was the average area per number of aggregates (A/N) at 48 hours after plating on Matrigel. The present inventors discovered large aggregates for AD patients when compared with age-matched controls for which cellular aggregation was more evenly distributed.

The prevent inventors developed a new diagnostic method(s) relating to cell aggregation. Disclosed herein is a method for diagnosing Alzheimer's disease based on quantitatively measured cellular aggregation rate. The present inventors discovered that the rate of change of cell aggregation with increasing cell density is elevated in AD cells compared to non-AD cells. The trend of higher rate of change of cell aggregation is consistent with the inventors' previous studies in which it was reported that AD cells are consistently bigger and less adhesive in average than the AC/Non-ADD cells.

Thus, disclosed herein is a method of diagnosing Alzheimer's Disease in a human subject comprising the steps of

(a) culturing one or more cells obtained from a human subject for a time period;

(b) determining an average area of cell aggregates and dividing the average area by the number of aggregates to obtain an average area per number of aggregates;

(c) determining an aggregation rate by evaluating the rate of change of the average area per number of aggregates determined in step (b) as a function of cell density, wherein the rate of change of the average per number of aggregates as a function of cell density is evaluated by determining the slope of a linear fit between the average area per number of aggregates and cell density;

(d) comparing the determination of step (c) with an aggregation rate determined using non-Alzheimer's disease cells; and

(e) diagnosing the presence or absence of Alzheimer's Disease based on the comparison in step (d), wherein the diagnosis is positive for Alzheimer's disease if the aggregation rate determined in step (c) is increased compared to the aggregation rate determined using the non-Alzheimer's disease cells.

In some embodiments, the method further comprises confirming the diagnosis using at least one additional diagnostic assay. In certain embodiments, the at least one additional diagnostic assay is chosen from AD-Index and Morphology diagnostic assays.

Also disclosed herein is a simple-majority rule for diagnosing AD using at least three diagnostic assays. There is disclosed a method of diagnosing Alzheimer's disease in a human subject comprising

i) performing at least three diagnostic assays;

ii) evaluating whether each assay indicates the presence or absence of Alzheimer's disease; and

iii) diagnosing the presence or absence of Alzheimer's disease based on whether a simple majority of the diagnostic assays indicate the presence or absence of Alzheimer's disease,

wherein one of the diagnostic assays comprises:

(a) culturing one or more cells obtained from a human subject for a time period;

(b) determining an average area of cell aggregates and dividing the average area by the number of aggregates to obtain an average area per number of aggregates;

(c) determining an aggregation rate by evaluating the rate of change of the area per number of aggregates determined in step (b) as a function of cell density, wherein the rate of change of the average per number of aggregates as a function of cell density is evaluated by determining the slope of a linear fit between the average area per number of aggregates and cell density; and

(d) comparing the determination of step (c) with an aggregation rate determined using non-Alzheimer's Disease cells, wherein the presence of Alzheimer's disease is indicated if the aggregation rate determined in step (c) is increased compared to the aggregation rate determined using the non-Alzheimer's Disease cells.

## BRIEF DESCRIPTION OF THE FIGURES

Figures 1(A) - (D) illustrate increased AD aggregation rate for banked skin samples compared to AC and Non-ADD;
Figures 1(E) and (F) plot slope and intercept in view of patient age from AD samples.
Figures 2(A) - (D) illustrate AD aggregation rates for samples from a clinic.
Figures 3(A) - (D) illustrate slope and intercept of A/N versus cell density; Figures 3(E) and (F) plot slope and intercept in view of patient age.
Figures 4(A) - (D) illustrate slope and intercept probability distributions.
Figures 5(A) and (B) illustrate average area of the unit aggregate (A/N) versus aggregation rate (slope).
Figures 6(A) - (F) illustrate cross-validation of aggregation rate with the AD-Index and Morphology assays.
Figures 7(A) and (B) illustrate a simple-majority rule by plotting AD-index values, morphology vales, and aggregation rates.
Figures 8(A) and (B) graphically illustrate quantification of cross-validation.
Figures 9(A) and (B) illustrate the test-retest reliability for the Aggregation Rate assay.

## DESCRIPTION

Abbreviations: AD: Alzheimer's disease; AC: non-demented age-matched control; Non-ADD: non-Alzheimer's dementia.

As used herein, the singular forms "a," "an," and "the" include plural reference unless the context dictates otherwise.

As used herein, "AD-Index" assay refers to a diagnostic assay for Alzheimer's disease based on the ratio of phosphorylated first and second MAP kinase proteins. The "AD-Index" assay comprises (a) contacting cells from a subject with an agent that is a protein kinase C activator; (b) measuring the ratio of a phosphorylated first MAP kinase protein to a phosphorylated second MAP kinase protein wherein the phosphorylated first and second MAP kinase proteins are obtained from the cells after the contacting in step (a); (c) measuring the ratio of phosphorylated first MAP kinase protein to phosphorylated second MAP kinase protein in cells from the subject that have not been contacted with the agent that is a protein kinase C activator used in step (a); (d) subtracting the ratio obtained in step (c) from the ratio obtained in step (b); and (e) diagnosing the presence or absence of Alzheimer's disease in the subject based on the difference calculated in step (d), wherein the presence of Alzheimer's disease is indicated in the subject if the difference is a positive value. U.S. Patent Number 7,595,167 and U.S. Patent Application Publication Number 2014/0031245 are referenced for their detailed disclosure of the AD-Index assay. Thus, the AD-Index assay may be performed as described in those publications. For example, in certain embodiments, the PKC activator is bradykinin and the first and second MAP kinase proteins are Erk1 and Erk2, respectively.

As used herein, "Morphology" assay refers to a diagnostic assay for Alzheimer's disease comprising (a) obtaining one or more cells from a human subject; (b) culturing the one or more cells for a time period; (c) determining the average area of cell aggregates and dividing the average area by the number of aggregates to obtain the average area per number of aggregates; and (d) comparing the determination of step (c) with the average area per number of aggregates determined using non-Alzheimer's disease cells, wherein the presence of Alzheimer's disease is indicated if the average area per number of aggregates determined in step (c) is greater than the average area per number of aggregates using the non-Alzheimer's disease cells. Cells may be cultured and the area and number of aggregates may be determined as described in U.S. Patent No. 8,658,134, which is referenced for its disclosure of the "Morphology" assay, and as described below.

As used herein, the term "simple majority" refers to an instance where a majority of diagnostic assays, for example, two out of three diagnostic assays, yield the same diagnosis (*i.e.* the presence or the absence of Alzheimer's disease).

The present inventors have discovered a new method for diagnosing AD based on an elevated cell aggregation rate with increasing cell density in cells from AD patients when compared with AC and Non-ADD patients. Thus, there is disclosed a method of diagnosing Alzheimer's Disease in a human subject comprising the steps of

(a) culturing the one or more cells for a time period;

(b) determining an average area of cell aggregates and dividing the average area by the number of aggregates to obtain an average area per number of aggregates;

(c) determining an aggregation rate by evaluating the rate of change of the average area per number of aggregates determined in step (b) as a function of cell density, wherein the rate of change of the average per number of aggregates as a function of cell density is evaluated by determining the slope of a linear fit between the average area per number of aggregates and cell density;

(d) comparing the determination of step (c) with an aggregation rate determined using non-Alzheimer's disease cells; and

(e) diagnosing the presence or absence of Alzheimer's Disease based on the comparison in step (d), wherein the diagnosis is positive for Alzheimer's disease if the aggregation rate determined in step (c) is increased compared to the aggregation rate determined using the non-Alzheimer's disease cells.

In some embodiments, the one or more cells obtained from the human subject are peripheral cells (*i.e.,* cells obtained from non-CNS tissue). In some embodiments, the one or more cells are fibroblast cells. In certain embodiments, the fibroblast cells are skin fibroblast cells. Cell precursors of fibroblasts, such as induced pluripotent stem cells (IPSC) may also be used. For example, recent techniques for obtaining IPSC from human skin fibroblasts permitted differentiation of IPSC in cells such as neurons and showed imbalances in Aβ in both skin fibroblasts and IPSC differentiated neurons. Whalley K., "Neurodegenerative disease: Dishing up Alzheimer's disease", Nature Reviews Neuroscience 13, 149 (March 2012) | doi:10.1038/nrn3201.

In some embodiments, the one or more cells are chosen from skin cells, blood cells (lymphocytes), and buccal mucosal cells.

The non-Alzheimer's disease cells may be chosen, e.g., from an age-matched control. In some embodiments, the age-matched control is chosen from a non-AD non-demented population. In some embodiments, the age-matched control is chosen from a non-AD demented population, such as patients with Huntington Chorea, B12 deficiency, or hypothyroidism.

The one or more cells may be cultured in a cell media for growth, such as, for example, a protein mixture. In some embodiments, the protein mixture is a gelatinous protein mixture. A non-limiting exemplary gelatinous protein mixture is Matrigel™. Matrigel™ is the trade name for a gelatinous protein mixture secreted by the Engelbreth-Holm-Swarm (EHS) mouse sarcoma cells and marketed by BD Biosciences. This mixture resembles the complex extracellular environment found in many tissues and is used by cell biologists as a substrate for cell culture.

In some embodiments, the one or more cells are cultured in a preparation comprising extracellular matrix proteins. In some embodiments, the preparation comprises laminin, collagen, heparin sulfate proteoglycans, entactin/nidogen, and/or combinations thereof. In some embodiments, the preparation is extracted from a tumor, such as the EHS mouse sarcoma. The preparation may further comprise a growth factor, such as TGF-beta, epidermal growth factor, insulin-like growth factor, fibroblast growth factor, tissue plasminogen activator, and/or other growth factors or combinations thereof. In certain embodiments, the growth factors occur naturally in the EHS mouse sarcoma. Extracellular matrix proteins may also contain numerous other proteins.

In some embodiments, the one or more cells are cultured in a basement membrane preparation. In some embodiments, the preparation is solubilized. In some embodiments, the basement membrane preparation is extracted from a tumor, such as the EHS mouse sarcoma a tumor rich in extracellular matrix proteins. Its major component is laminin, collagen IV, heparin sulfate proteoglycans, and entactin/nidogen. In some embodiments, the preparation contains TGF-beta, epidermal growth factor, insulin-like growth factor, fibroblast growth factor, tissue plasminogen activator, and/or other growth factors which may or may not occur naturally in the EHS tumor. BD Matrigel Matrix Growth Factor Reduced (GFR) is found to be particularly well suited for applications requiring a more highly defined basement membrane preparation.

Within a short time after being cultured, measurable cellular networks form. This time period may vary in view of, for example, cell type and conditions, but generally, this time period ranges from about 1 hour or less, ranging from about 10 minutes to about 60 minutes, such as from about 10 minutes to about 45 minutes or any time in between. After a time, for example, approximately 5 hours, these networks start to degenerate and edges retract to leave behind measurable "clumps" or aggregates. In some embodiments, the time period for culturing the one or more cells is chosen from about 1 hour to about 72 hours, such as from about 12 hours to about 72 hours or from about 24 hours to about 48 hours. In certain embodiments, the time period is about 48 hours or any one hour increment subdivision thereof.

The method may further comprise imaging the cultured cells at the end of the time period. Images may be captured according to techniques known in the art. For example, images of the cellular networks may be captured with an inverted microscope, such as Western Digital AMID Model 2000, and controlled by a computer via image acquisition software at a desired magnification. Appropriate imaging techniques include, but are not limited to, confocal microscopy, phase contrast, bright field, fluorescence, differential interference contrast, and robotic systems.

The area of aggregates can be determined by any suitable method, e.g., by fitting an ellipse across the aggregate. The counting of aggregates as well as aggregate area determination can be performed manually or can be automated, e.g., by image processing techniques known in the art.

In some embodiments, cell density is measured based on the number of cells per $\mu m^2$ or per field of view. In certain embodiments, cell density is measured by measuring the number of cells per 10× image. In certain embodiments, the rate of change of the average area per number of aggregates as a function of cell density is evaluated within the boundaries of 320 to 550 cells/10× image or such as, of 330 to 500 cells/10x image.

Cell aggregation rate is determined by evaluating the rate of change of the average area per number of aggregates as a function of cell density. The rate of change of the average area per number of aggregates as a function of cell density is evaluated by determining the slope of a linear fit between the average area per number of aggregates and cell density.

The aggregation rate of the cultured cells obtained from the human subject is compared to the aggregation rate determined using non-Alzheimer's disease control cells. The diagnosis is positive for Alzheimer's disease if the aggregation rate of the cultured cells from the human subject is increased compared to the aggregation rate determined using the non-Alzheimer's disease control cells.

The AD diagnosis may be confirmed by at least one additional diagnostic assay. In some embodiments, the at least one additional diagnostic assay is performed using cells obtained from the human subject. That is, the Aggregation Rate assay and the at least one additional diagnostic assay use the same cell lines, *i.e.,* patients. In certain embodiments, the at least one additional diagnostic assay is chosen from AD-Index and Morphology diagnostic assays. In some embodiments, the diagnosis is confirmed by both the AD-Index and Morphology assays. The Aggregation Rate assay may also be combined with other diagnostic technologies known in the art, including, but not limited to, clinical diagnosis, positron emission tomography (PET) - amyloid imaging, and/or cerebrospinal fluid (CSF) levels of Aβ or tau.

In some embodiments, an AD diagnosis is based on a simple majority of at least three diagnostic assays. Thus, there is disclosed a method of diagnosing AD in a human subject using an aggregation rate, such as comprising: i) performing at least three diagnostic assays; ii) evaluating whether each assay indicates the presence or absence of AD; and iii) diagnosing the presence or absence of AD based on whether a simple majority of the at least three diagnostic assays indicate the presence or absence of AD, wherein one of the diagnostic assays comprises: a) culturing one or more cells obtained from a human patient for a time period; b) determining an average area of cell aggregates and dividing the average area by the number of aggregates to obtain an average area per number of aggregates; c) determining an aggregation rate by evaluating the rate of change of the area per number of aggregates determined in step (b) as a function of cell density, wherein the rate of change of the average per number of aggregates as a function of cell density is evaluated by determining the slope of a linear fit between the average area per number of aggregates and cell density; and d) comparing the determination of step (c) with an aggregation rate determined using non-AD cells, wherein the presence of AD is indicated if the aggregation rate determined in step (c) is increased compared to the aggregation rate determined using the non-AD cells.

In some embodiments, two of the at least three diagnostic assays are AD-Index and Morphology assays.

The methods described herein will be further described by the following examples, which are meant to illustrate, but not limit, the scope of the disclosure.

## EXAMPLES

### *Materials and Methods*

**Banked and Fresh Cell Lines Used in This Study.** Example experiments were carried out using skin fibroblast samples from 38 patients 9 banked cases (Supplementary Table 1) provided by the Coriell Institute for Medical Research (Camden, NJ), and 29 cases (Supplementary Table 2) from the clinic provided by Marshall University (Huntington, WV). The analysis showed a clear separation between AD and AC/Non-ADD using cellular aggregation rate as a diagnostic tool.

The fibroblast cells were plated on a thick layer (~1.8 mm) of 3-D matrix (Matrigel, BD Biosciences, San Jose, CA) on 12 well plates. *See* Chirila F.V. et al., "Spatiotemporal Complexity of Fibroblast Networks Screens for Alzheimer's Disease," J. Alzheimer's Disease, 33, 165-176 (2013). The available patient information is posted on Coriell web site (http://ccr.coriell.org/). The cell lines analyzed (38) were largely from the clinic (29/38) serving also as a validation for the previous studies with banked samples. *See id.* The age-matched control (AC) cases were not demented at the date of skin biopsy extraction. All the samples were taken antemortem. The banked skin fibroblast cells were frozen stocks under liquid nitrogen. Primary cultures were established after thawing those frozen samples and followed through successive passaging. See Zhao W. Q. et al., "MAP kinase signaling cascade dysfunction specific to Alzheimer's disease in fibroblasts," Neurobiol. Dis., 11, 166-183 (2002); Khan T. K. et al., "An internally controlled peripheral biomarker for Alzheimer's disease: Erk1 and Erk2 responses to the inflammatory signal bradykinin," Proc. Natl. Acad. Sci. U.S.A., 103(35), 13203-7 (2006); Khan T. K. et al., "Early diagnostic accuracy and pathophysiologic relevance of an autopsy-confirmed Alzheimer's disease peripheral biomarker," Neurobiol. Aging, 31(6), 889-900 (2008); Khan T. K. et al., "A cellular model of Alzheimer's disease therapeutic efficacy: PKC activation revesres Abeta-induced biomarker abnormality on cultured fibroblasts," Neurobiol. Dis. 34(2), 332-9 (2009); Chirila F. V. et al., "Spatiotemporal Complexity of Fibroblast Networks Screens for Alzheimer's Disease," J. Alzheimer's Disease, 33, 165-176 (2013). All cell lines used were primary cell lines and were not treated in order to be immortalized.

Freshly taken fibroblasts were obtained as follows. Punch-biopsies (2-3 mm, upper arm) of skin tissues from patients and controls were obtained. The method of isolating fibroblasts from skin biopsies was followed according to the protocol in Takashima, A., "Establishment of Fibroblast Cultures," Current Protocols in Cell Biology, vol. 2, 2.1.1-2.1.12 (1998). Cells with passages between 5 and 15 were used.

The initial cell density was controlled to be 50 cells/mm$^3$ and was homogenized with 1.5 ml Dulbecco Modified Eagle Medium with 10% fetal bovine serum and 1% penicillin/streptomycin for each well. Cells were kept in a $CO_2$ water-jacket incubator (Forma Scientific) up to 7 days after plating.

**Image Capture.** Images of the cellular networks were captured with an inverted microscope (Westover Digital AMID Model 2000, Westover Scientific, Bothell WA), controlled by a computer via an image acquisition software (Micron 2.0.0), using a 10x and a 4x objective. Five to nine images captured per well and four wells per cell line were typically used. In the first day, images were acquired at 30 min after seeding, the second day at 24 hours, and the third day at 48 hours. Images were processed with ImageJ, a freely available software from NIH (http://rsbweb.nih.gov/ij/).

The 5 images per well were initially taken using the same standard pattern, center (1), up (2), down (3), left (4), right (5), by moving one image field with respect to the central image. See Chirila F. V. et al., "Spatiotemporal Complexity of Fibroblast Networks Screens for Alzheimer's Disease," J. Alzheimer's Disease, 33, 165-176 (2013). Later in the process, the number of images was increased from 5 to 9 by filling the corners of the rectangle with images from 6 to 9, in order to increase the area investigated and further improve the coefficient of variation without affecting the diagnostic discriminability. *See id.* Image 1 was always in the center of the well. To determine the center of the well, one of the following methods was used: **a)** the live image under $4\times$ magnification should be symmetric, i.e., the shadows in the four corners should have equal areas for an aligned microscope; **b)** mark the center with a needle; or **c)** use gridded plates (Pioneer Scientific; Shrewsbury, MA) where the central square is always the 6th, in the central row or column. For image quantification, two sets of tools were used: initially manual as provided by Micron, software which came with the microscope; later automated with ImageJ.

For the initial cell count, a custom ImageJ plug-in was used in which "despeckle" was run three times; the image was filtered three times with a minimum filter of radius 0.5; and "Subtract Background" was run with a rolling radius of 20. Finally, the image was made binary and "Analyze Particles" was run in the size range 200-10000. All of these ImageJ commands were run inside a loop to permit analysis of all the images from one cell line automatically. The ImageJ plug-in was tuned by using manual cell counts on the same images and the relative error was below 7%.

The target number of cells per 10x image was 417 which corresponded to an initial cell concentration of 50 cells/ml. See Bikfalvi A. et al., "Phenotypic modulations of human umbilical vein endothelial cells and human dermal fibroblasts using two angiogenic assays," Biol. Cell, 72, 275-278 (1991). A variation of cell concentration between 45 and 60 cells/ml was permitted. To minimize heterogeneity of the cell distribution in the image, images outside of the range 320-550 cells per $10\times$ image were eliminated. For cellular aggregates at 48 h, manual ellipse fitting with the Micron software was used.

**Average area per number of aggregates (A/N).** Average area per number of aggregates was calculated in the following manner. For each image (i), an average aggregate area, <A>$_i$, was calculated and the number of aggregates, $N_i$, was counted. Then, for each image, the ratio <A>$_i$/$N_i$ was evaluated. Typically, nine images per well were used and an average area per number for each well was evaluated as the

$$\langle\langle A\rangle_i/N_i\rangle = \frac{1}{9}\sum_{i=1}^{9}\frac{\langle A\rangle_i}{N_i}.$$

An addition average was performed over the four wells:

$$\langle\langle\langle A\rangle_i/N_i\rangle\rangle = \frac{1}{4}\sum_{w=1}^{4}\left(\frac{1}{9}\sum_{i=1}^{9}\frac{\langle A\rangle_i}{N_i}\right)_w.$$

For simplicity, A/N is used instead of $\langle\langle\langle A\rangle_i/N_i\rangle\rangle$ and A/N is called area of the unit aggregate. The aggregates were manually fitted with ellipses using Micron 2.0 software and their area and number were recorded. An automatic script for ImageJ was developed which agreed well with the manual ellipse fitting. These two approaches were within one standard deviation of each other.

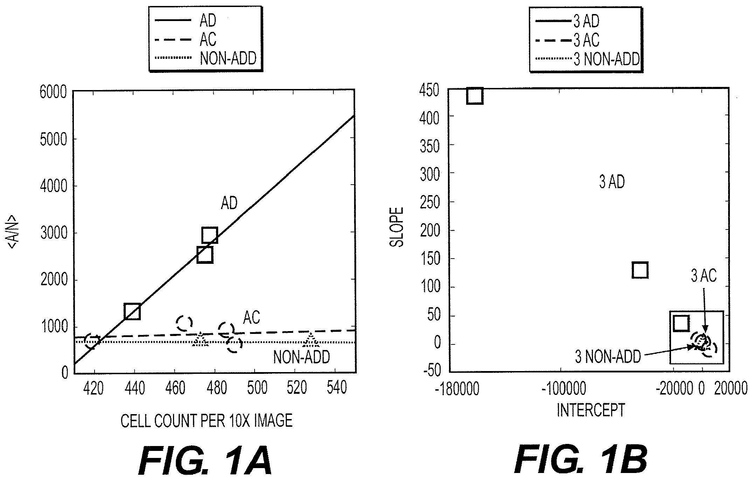

**Aggregation rate.** The dependence of A/N on cell density was evaluated (**Fig. 1A**; Fig. 1A plots the dependence of area per number of aggregates (A/N) on the cell density (# cells/10x image field), where AD cases are squares, AC cases are dashed circles, and Non-ADD cases are dotted triangles), and the dependence was fit with a line, $f(6x)=s*x+int$. From the linear fit, the slope(s) and intercept (int) for the population of 38 cases were evaluated. The linear fit was performed within the boundaries 320 to 550 cells per 10x image field.

For data analysis, Gnuplot 4.4, freely available software (http://www.gnuplot.info), was used. A built in fit function from Gnuplot, which used an implementation of the nonlinear least-squares (NLLS) Marquardt-Levenberg algorithm, fit the raw data points. Unless otherwise specified, the error-bars are standard errors of the mean (SEM).

**Probability distribution of cellular aggregates.** For all the AD and AC patients, the values for slope(s) and intercept (int) of aggregates were binned into equal intervals, fit with Gaussian functions for each variable, and then integrated into a normalized two-dimensional distribution.

**Example 1: Increased AD Aggregation Rate for Banked Samples**

As shown in the inventors' previous studies, cell aggregation is enhanced in Alzheimer's disease (AD) patients when compared with age-matched controls (AC) and non-Alzheimer's disease demented patients (Non-ADD). See Chirila F. V. et al., "Spatiotemporal Complexity of Fibroblast Networks Screens for Alzheimer's Disease," J. Alzheimer's Disease, 33, 165-176 (2013). Cell aggregation was previously quantified by the area of the unit aggregate, which was the average area per number of aggregates (A/N) at 48 hours, after plating on Matrigel. See id. Here, a new way of screening Alzheimer's disease patients (AD) from age-matched controls (AC) and from non-Alzheimer's disease demented patients (Non-ADD) is based on the inventors' discovery of increased cell aggregation when the cell density increases, referred to here as the slope intercept representation of cell aggregation.

An example of the dependence of cell aggregation (A/N) on cell density (# cells/10× image field) is illustrated in **Fig. 1A** for the banked samples. The dependence was studied in the cell density range of 320 to 550 cells per 10x image field. The Alzheimer's disease (AD) cases are denoted by squares, the age-matched control (AC) cases by dashed circles, and the non-Alzheimer's disease demented cases (Non-ADD) by dotted triangles. The linear dependence for the three examples--AD, AC, and Non-ADD is illustrated by the fit lines (**Fig. 1A**). The AD (square data points) slope is steeper than the AC (circle data points) slope or Non-ADD (triangle data points) slope, and, as a consequence, the AD intercept is a lot more negative than the intercept for the AC and Non-ADD (**Fig. 1A, B**; Fig. 1B plots the slope and intercept of the cases, indicating a higher slope and a more negative intercept for the AD cases compared to AC and Non-ADD).

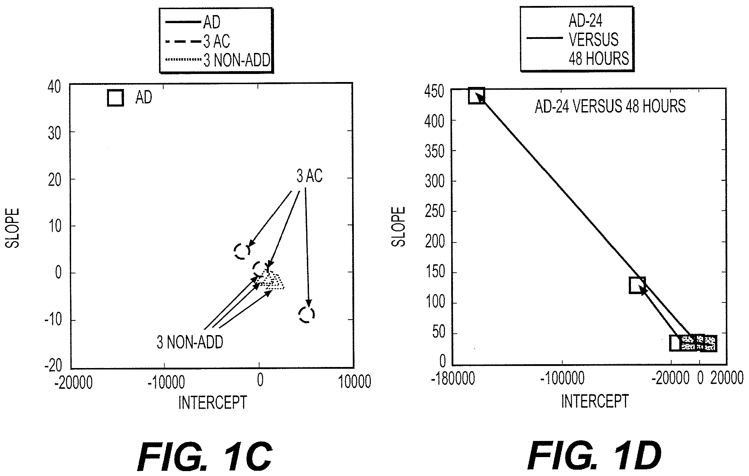

A small number of banked cases from Coriell (9 cell lines)--3AD, 3AC, and 3 Non-ADD showed the same separability between AD and the other two groups (**Fig. 1B, C**; Fig. 1C shows a zoomed-in view of the rectangle in **(B)**, showing a significant gap in slope and intercept between the lowest AD case and upper AC case). The line fit was done in the window 320-550 cells per 10x image. A zoom in for the rectangle in **Fig. 1B,** presented in **Fig. 1C,** shows a gap both in the slope (~30) and the intercept (15000), between the near cut-off AC case and the near cut-off AD case.

The data also showed that the AD-AC/Non-ADD separation increased for the 48-h time point when compared with the 24-h time point (**Fig. 1D**; Fig. 1D plots the slope and intercept for the AD cases at 48 h (empty symbols) compared to at 24 h (filled symbols), showing a steeper slope and more negative intercept at 48 h). The arrows indicate that for all 3 AD cases the slope is steeper and therefore the intercept is more negative at 48 h when compared with the 24-h time point. In other words the AD cases moved further away from the cut-off at 48 h when compared with 24-h, indicating that 48 h is an optimum time-point for this biomarker.

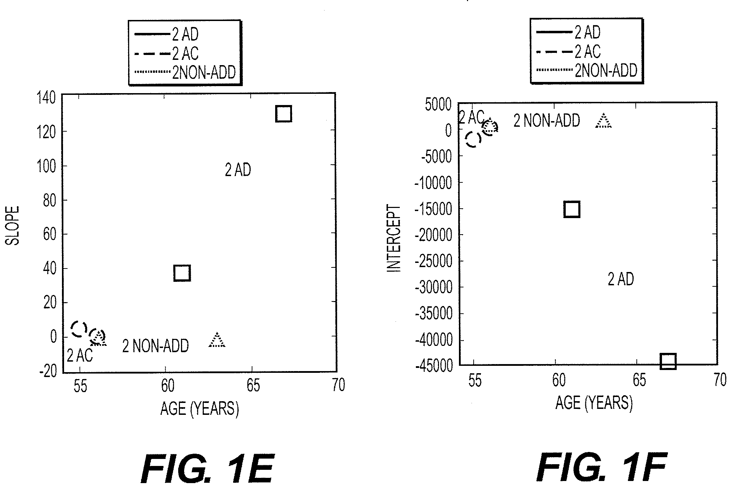

The aggregation rate (slope) and intercept changed with age as depicted in **Fig. 1E** (Fig. 1E plots slope dependence on the age of the patient in the age range 55 to 70 years, indicating a higher aggregation rate for AD cases in this range) and **Fig. 1F** (Fig. 1F plots the dependence of the intercept on the age of the patient for the same cases shown in **(E)**), and, for approximately the same age-range (55-70 years), the AD cases showed a higher aggregation rate. Previous studies of the dependence of cell aggregation (A/N) on age showed that the AD diagnostic discriminability is preserved in the age range of 50-90 years.

The data showed that the slope intercept representation for cell aggregation is a more refined representation of cell aggregation than a simple average, i.e., A/N. It may be a useful tool to resolve the AD/AC/Non-ADD cases which are too close to the cut-off line in the A/N representation. Typically, the cut-off line for the AD and AC populations is at the intersection of the two Gaussian distributions that fit the biomarker outputs. In a narrow region near the but-off line, the tails of the Gaussian distributions coexist with certain probability therefore defining a gray zone of false positive/negative. Unknown cases falling in the gray zone for the A/N measure might be removed by using the rate of change of A/N.

**Example 2: Validation of Increased AD Aggregation Rate with Fresh Samples from the Clinic**

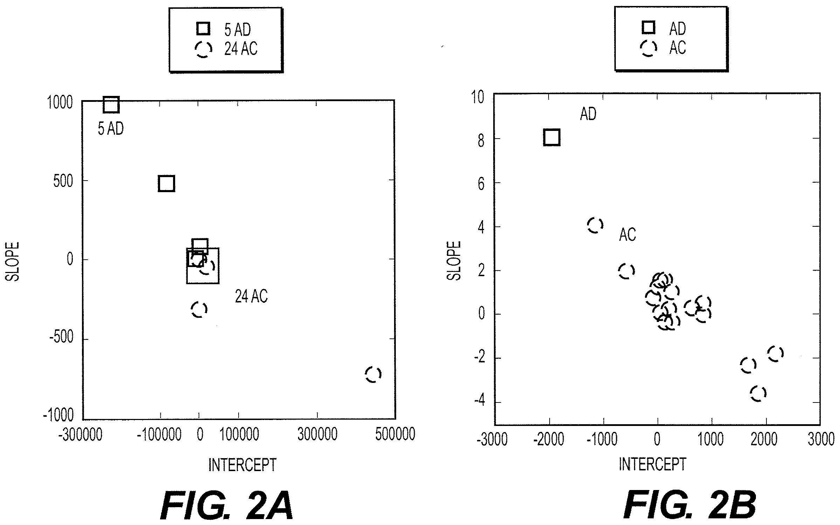

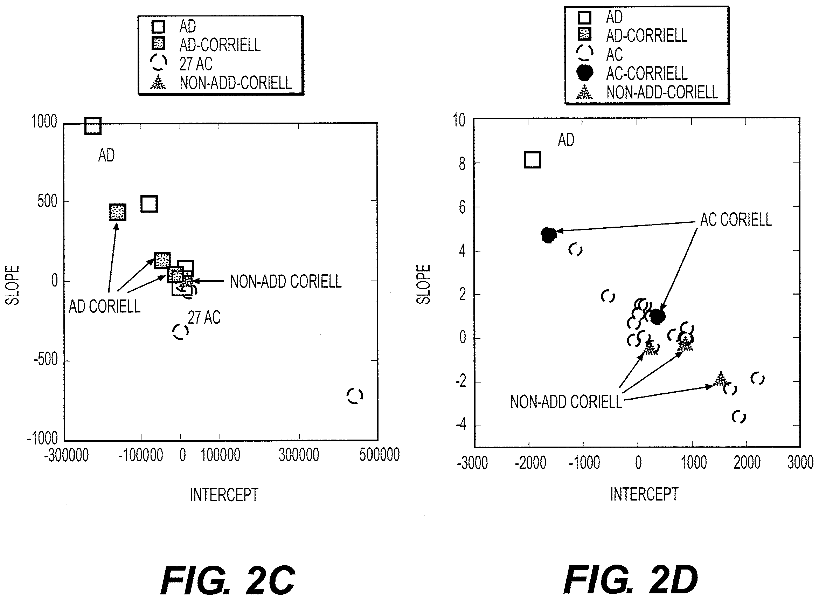

The discrimination of AD cases from AC cases using the slope intercept analysis was further investigated on the 29 samples from the clinic. Among these samples, 5 were AD cases and 24 were AC cases. A higher slope and more negative intercept for the AD cases was confirmed (**Fig. 2A**; Fig. 2A plots slope and intercept for 24 AC and 5 AD samples from the clinic), as well as the gap between the AD and AC groups (**Fig. 2B**; Fig. 2B shows a zoomed-in view near the cut-off (rectangle in **(A)**), revealing a gap in the slope as well as in the intercept between AD and AC). The slope and intercept for the banked samples and fresh samples showed the same trend (**Fig. 2C;** Fig. 2C plots slope and intercept of banked samples (filled symbols) and clinic samples (empty symbols), showing similar trends and separation between AC and AC groups). The size of the gap in slope (~2) and intercept (~500) were also preserved when these two data-sets were plotted (**Fig. 2D**; Fig. 2D shows a zoomed-in view near the cut-off (rectangle in **(C)**). Narrowing of the gaps in slope and intercept was due to population increase from the 9 banked cases to 38 cases overall, which included the 29 samples from the clinic.

**Example 3: Aggregation Rate Drives the Separation between the AD and AC Groups**

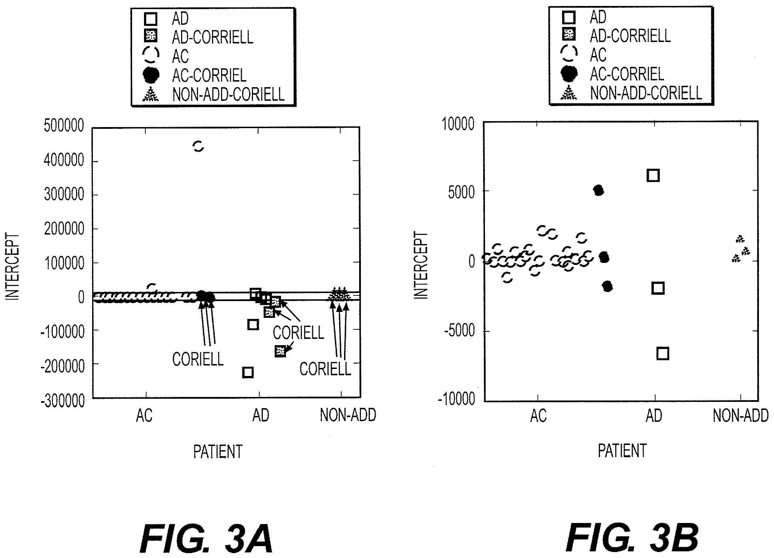

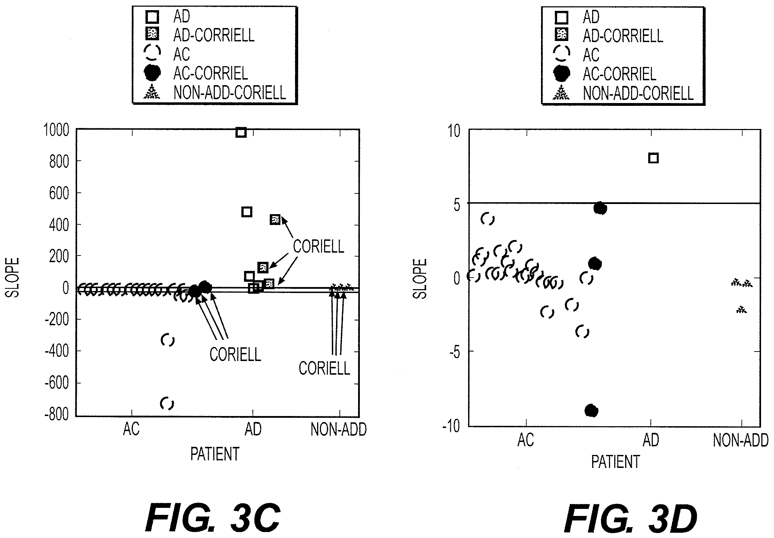

The slope and intercept of A/N versus cell density were analyzed independently. Looking at the intercept for the AC, AD and Non-ADD groups both at large scale (**Fig 3A**; Fig. 3A plots intercept for 24 AC and 5 AD samples from the clinic (empty symbols) and 3 AC, 3 AD, and 3 Non-ADD banked samples (filled symbols) as well as when zoomed in near the cut-off locus (**Fig. 3B**; Fig. 3B shows a zoomed-in view near the cut-off (rectangle in **(A)**), revealing overlapping values in the intercept), an overlap was observed. The slope, i.e. the rate of change, however, showed a distinct separation between AD and AC/Non-ADD groups (**Fig. 3C, D**; Fig. 3C plots slope for the same samples as in **(A);** Fig. 3D shows a zoomed-in view near the cut-off (rectangle in **(C),** revealing no overlapping values in the slope). Thus, the driving force of the separation observed between the AD and AC/Non-ADD groups was the rate of change of cell aggregation with increasing cell density.

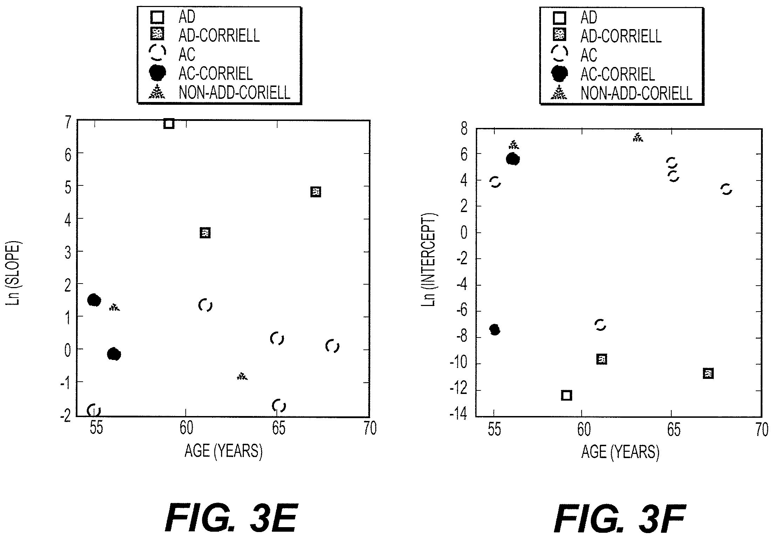

As shown in **Fig. 1E** and **Fig. 1F,** the aggregation rate (slope) and intercept change with age as depicted in **Fig. 3E** (Fig. 3E plots slope dependence on the age of the patient in the age range 55-70 years, showing a higher aggregation rate for AD cases in this range) and **Fig. 3F** (Fig. 3F plots dependence of the intercept on the age of the patient for the same cases as in **(E)**), and for approximately the same age-range, 55-70 years, the AD cases showed higher aggregation rate. The intercept was more negative for the AD cases than for the AC and Non-ADD cases. **Figs. 3E** and **3F** use the natural logarithm of the actual values, and to show negative values, -ln(-negative values) was used.

**Example 4: Slope and Intercept Probability Distributions**

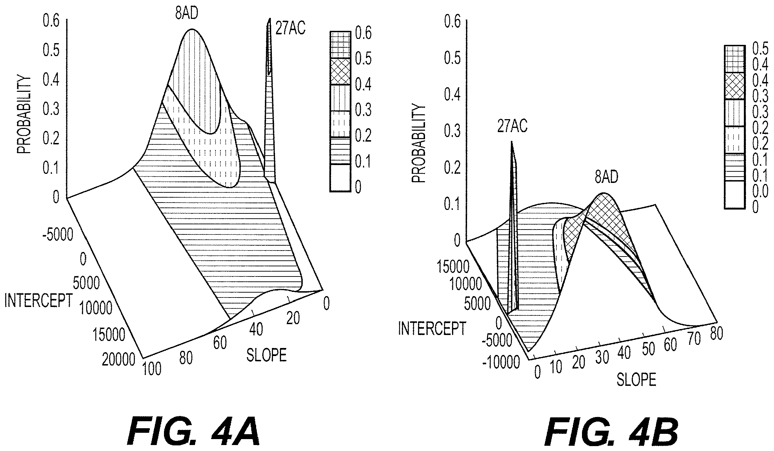

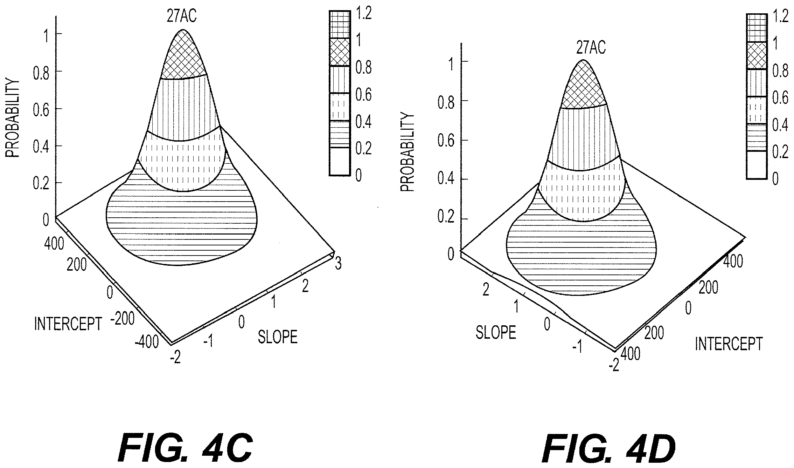

The value of an AD bio-marker can be assessed by using the probability distributions for the two groups, AD and AC. Based on the probability distributions, one can estimate the extent of possible overlapping probability. The two groups of data (8 AD, 27 AC) were binned in slope and intercept. The probability distributions were estimated based on the frequency of occurrence in each bin divided by the total number of occurrences. The raw probability data sets were fitted with Gaussian curves in variable, slope and intercept. Then, the two dimensional Gaussian probabilities were plotted using Gnuplot. The AC probability distribution was narrower than the AD probability distribution (**Fig. 4A, B**; Fig. 4A shows probability distributions for 8 AD and 27 AC; Fig. 4B shows another view for the probability distribution for 8 AD and 27 AC). The AC probability distribution resided on the tail of the AD probability distribution (**Fig. 4C, D**; Fig. 4C shows probability distribution for the 27 AC subjects, revealing that an overlapping probability with the AD group is possible for larger data sets, and is less than 10%; Fig. 4D shows another view for the probability distribution for the 27 AC, enforcing the small overlapping probability with the AD group) which accounted for a slight upward shift (<10%) in the baseline. This upward shift was also the overlapping probability which was <10%. Although the data from 27 AC and 8 AD cases did not overlap in the slope variable, the data suggested a possible overlap for larger data sets. The estimated overlapping probability was less than 10%.

**Example 5: Average Area per Number of Aggregates versus the Aggregation Rate**

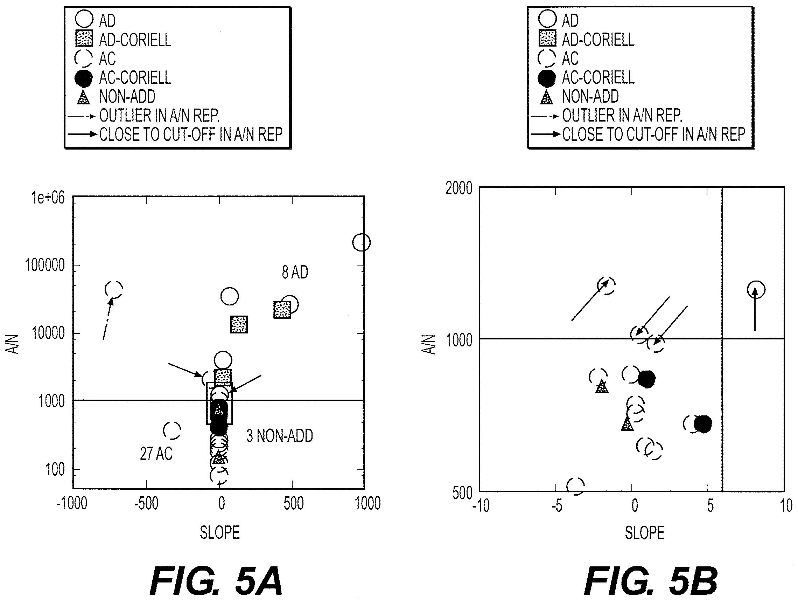

The population of 38 cases (8 AD, 27 AC, and 3 Non-ADD) were evaluated from the point of view of the efficacy of the two bio-markers quantified by the average area of the unit aggregate (A/N) and aggregation rate (slope). The A/N measure of cell aggregation was plotted against the aggregation rate (slope) in **Fig. 5.** In the A/N representation (y axis), one AC case was marked as an outlier because it was above the horizontal line which is the cut-off line (A/N=1000). (**Fig. 5A**; Fig. 5A plots A/N versus slope for the 38 cases, AD=squares and solid empty circles, AC=dashed empty circles and solid filled circles, Non-ADD=triangles, filled symbols=banked samples, empty symbols=clinic sample. The long-dash-dot arrow points to an AC outlier in the A/N representation, but which is screened out correctly in the slope representation. Solid arrows indicate AC/AD cases too close to the cut-off line in the A/N representation, but which are screened out correctly in the slope representation). The zoom in of the rectangular area from **Fig 5A** revealed that 3 AC cases and 1 AD case were too close to the cut-off line. (**Fig 5B;** Fig. 5B shows a zoomed-in view for the rectangle from **(A),** revealing that the 3 AC (arrows) and 1 AD (arrow) cases which were too close to the cut-off line in the A/N representation were screened out correctly in the slope representation). Therefore, in this population of 38 cases, 5 cases were uncertain in the A/N representation, with 1 case as an outlier and 4 cases too close to the cut-off line (gray zone). However, using the aggregation rate representation (x axis) of the cell aggregation, the uncertainty for the 5 cases was removed. The 4 AC cases were on the left side of the vertical cut-off line, and one AD case was at the right side of the vertical cut-off line (**Fig. 5B**), therefore diagnosed the same as the clinical diagnosis. Thus, the rate of change of cell aggregation with increasing cell density is a powerful tool for checking the less sophisticated measure of average area per number of aggregates (A/N).

**Example 6: Cross-Validation**

AD is a complex disease with a multifactorial structure in which many pathways are disrupted and/or affected. Furthermore, the sporadic form of AD has an onset above 65 years old when the likelihood of co-morbidity with other diseases is very high. Therefore, a single bio-marker may be unlikely to detect with high precision sporadic AD cases in their early progression when the drug efficacy for AD might be very high. The disclosed biomarker quantifying the fibroblast aggregation rate, when cross-validated with other two more mature biomarkers, AD-Index and Morphology assays, showed an overlap of approximately 92%.

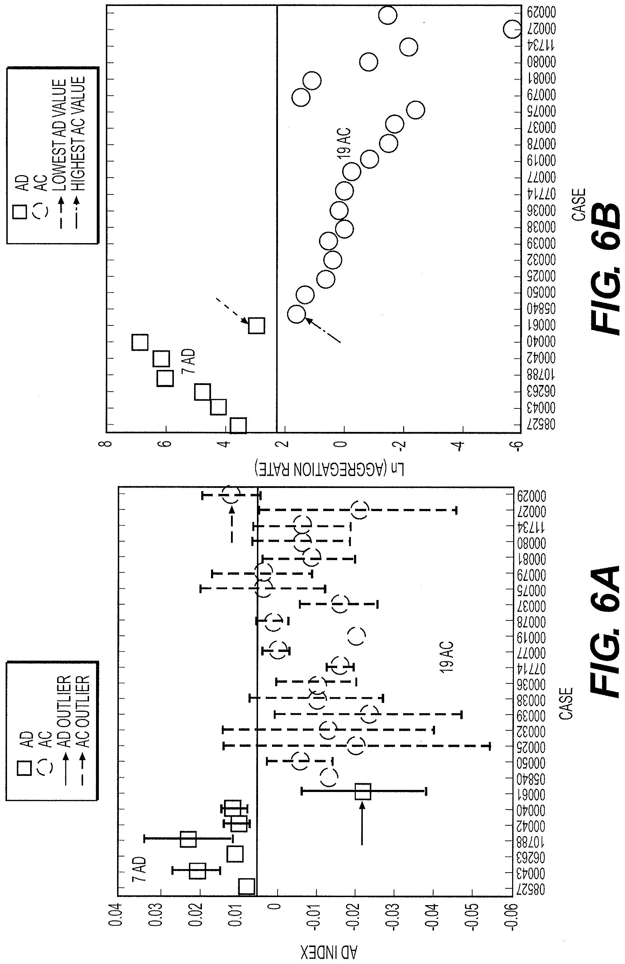

**Cross-validation of the aggregation rate with the AD-Index.** A comparison of the aggregation rate with a developed assay such as the AD-Index can be a measure of performance. See Hadley MA et al., "Extracellular matrix regulates Sertoli cell differentiation, testicular cord formation and germ cell development," J. Cell Biol., 101, 1511-1522 (1985); Ingber D. et al., "Mechanochemical switching between growth and differentiation during fibroblast growth factor-stimulated angiogenesis in vitro. Role of extracellular matrix," J. Cell Biol., 109, 317-331 (1989); Furukawa KS et al., "Tissue-engineered skin using aggregates of normal human skin fibroblasts and biodegradable material," J. Artif. Organs, 4, 353-356 (2001); Furukawa KS et al., "Formation of human fibroblast aggregates (spheroids) by rotational culture," Cell Transplantation, 10, 441-445 (2001). Out of the 38 cases tested with the aggregation rate assay, 26 were also tested with the AD-Index assay western blot. Out of these 26 cases tested with both assays, 24 gave the same diagnosis, representing 92.3% overlap between the two biomarkers (**Fig. 6A, B;** Fig. 6A plots the AD-Index assay for each of the 26 cases (AD=squares, AC=dashed empty circles), where the two outliers (1 AD, 1 AC) are indicated by the respective arrows; Fig. 6B plots the natural logarithm of the aggregation rate for each of the same 26 cases as in **(A),** where the respective arrows show the lowest AD value and the highest AC value). As the AD-Index is a well developed and tested assay on more than 80% hyper-validated samples, it is considered as a standard for the comparison with the aggregation rate. The aggregation rate agreed 100% with the clinical diagnosis for this population of 26 cases.

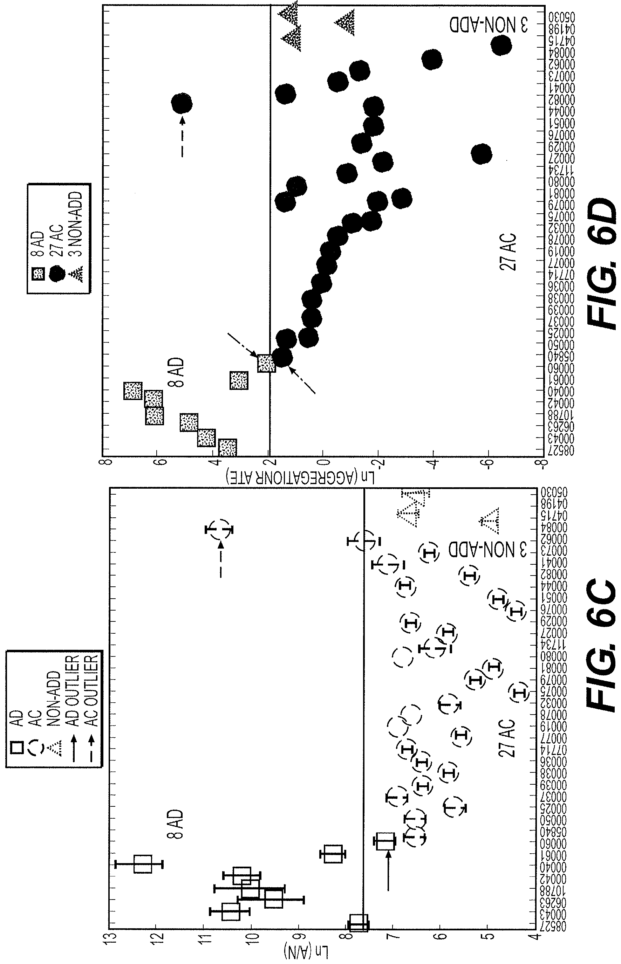

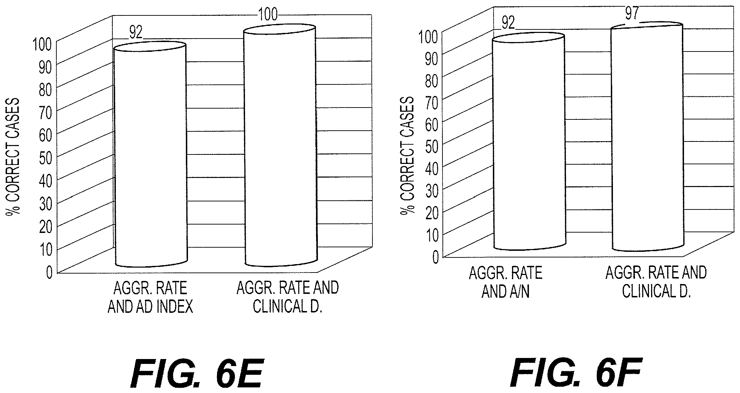

**Cross-validation of the aggregation rate with the Morphology assay.** A comparison of the aggregation rate with the Morphology assay, a studied assay, can also be a measure of performance. *See* Chirila F. V. et al., "Spatiotemporal Complexity of Fibroblast Networks Screens for Alzheimer's Disease," J. Alzheimer's Disease, 33, 165-176 (2013). All of the 38 cases tested with the aggregation rate were also tested with the simpler assay of average area per number of aggregates (A/N). Out of these 38 samples tested with both assays, 35 gave the same diagnosis, representing 92.1% overlap between the two bio-markers (**Fig. 6C, D**; Fig. 6C plots Ln(A/N) for each of the 38 cases, where the two outliers (1 AD, 1 AC) are indicated by the respective arrows; Fig. 6D plots the natural logarithm of the aggregation rate for each of the same 38 cases as in **(C);** the horizontal lines in **(A)-(D)** represent the cut-off for the AD-Index, Ln(Aggregation Rate), and Ln (A/N)). As A/N is a more developed and tested assay on more than 80% hyper-validated samples, it was considered as a standard for the comparison with the aggregation rate. For this population of 38 cases, the aggregation rate agreed in 97% of the cases with the clinical diagnosis. **Fig. 6E** shows the percent of overlap between the Aggregation Rate assay and the AD-Index assay, and the Aggregation Rate assay and clinical diagnosis, for the 26 cases diagnosed. **Fig. 6F** shows the percent overlap between the Aggregation Rate assay and the Morphology assay, and the Aggregation Rate assay and clinical diagnosis, for the 35 cases diagnosed.

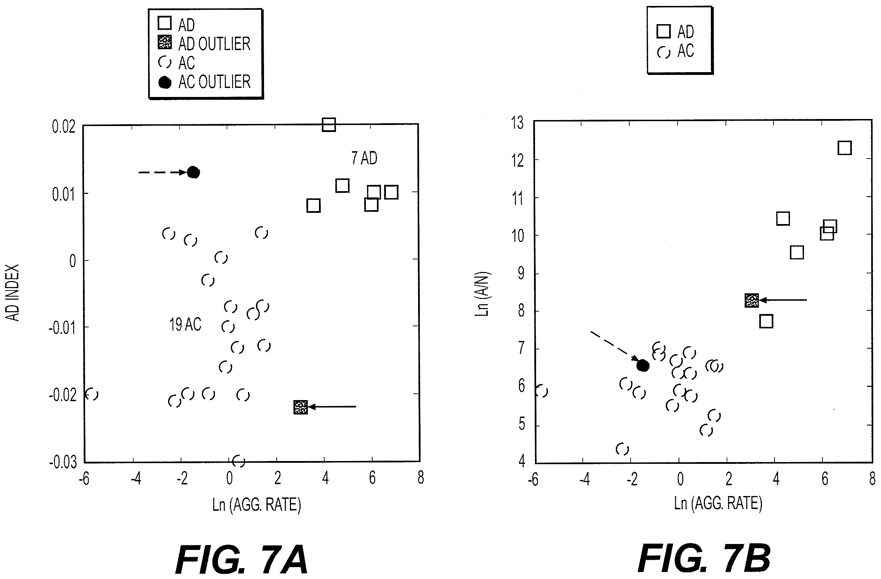

**Simple-majority rule.** Considering the 26 cases that were tested with all three bio-markers and upon use of a simple majority rule, i.e. 2/3 assays give the same result, the agreement with the clinical diagnostic increased to 100% (**Fig. 7**). As shown in **Fig. 7A,** two cases, 1 AD and 1 AC were outliers in the AD-Index assay. (Fig. 7A plots AD-Index versus the Ln(Aggregation Rate), revealing two cases, 1 AD (filled square and arrow) and 1 AC (filled circle and arrow), as outliers for the AD-Index assay). But as shown in **Fig. 7B** (Fig. 7B plots Ln(A/N) versus Ln(Aggregation Rate), the two outliers for the AD-Index were not outliers for the Morphology assay and Aggregation Rate assay, i.e., logarithm of the area per number of aggregates versus the logarithm of the aggregation rate, resulting in 100 % agreement with the clinical diagnosis. Thus, using more than one assay for diagnosing AD, as well as cross-validation using a majority rule, may improve the rate of success for final diagnosis. The method of cross-validation of the three assays can also increase the confidence of the clinical diagnosis. The same simple majority rule is expected to hold when used with reference to hyper-validated samples, i.e., autopsy confirmed AD and Non-ADD and non-demented AC.

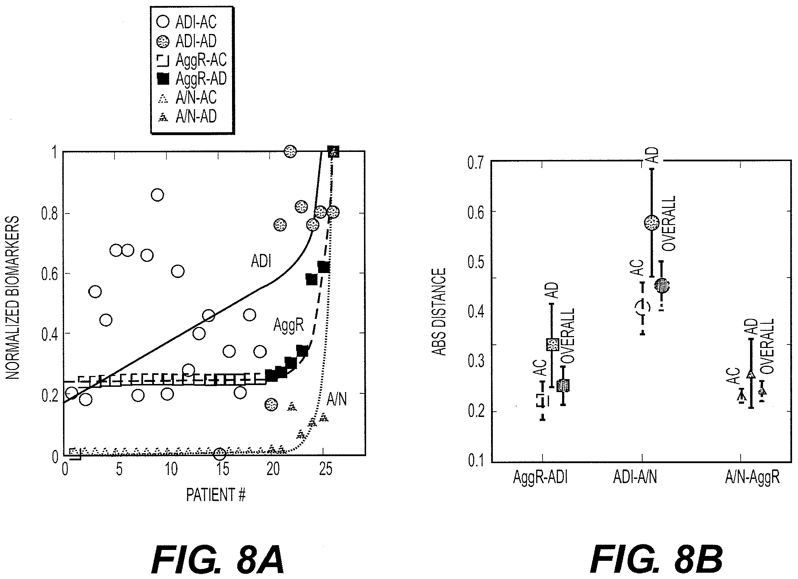

**Quantification of cross-validation and majority rule.** The three biomarker values were normalized between 0 and 1 as

$$\frac{(x-min)}{(max-min)},$$

where x was the biomarker value to be normalized, max was the maximum value, and min was the minimum value of the data set of 26 cases. This normalization brought all three biomarkers within the same range, 0 to 1, making the comparison easier (**Fig. 8A**).

The curve fitting for all three biomarker values was done in two steps. First the fitting was done with a linear function, f(x)=a*x+b, for the AC values and with an exponential function, g(x)=c*exp(d*x), for the AD values. Second, the fitting was done with a glued function h(x)= f(x) + g(x)= a*x+b + c*exp(d*x). For the starting values of the parameters a, b, c, and d, the end values from the previous fit were used. After fitting with the glued function, h(x), the new fit parameters were recorded and used for the plots in **Fig. 8A** (Fig. 8A shows normalized biomarkers between 0 and 1, where Aggregation Rate=AggR (squares), AD-Index=ADI (circles), and A/N=A/N (triangles), for 26 cases (19 AC=empty symbols, 7 AD=filled symbols).

The absolute difference per case, $|b_1-b_2|$, $|b_2-b_3|$, and $|b_3-b_1|$ where $b_1$=AggR, $b_2$=ADI, $b_3$=A/N, were used to assess which pair of biomarkers was best correlated. The average values of the absolute differences were then calculated

$$\langle |b_j - b_k| \rangle_{AC} = \frac{1}{19} \sum_{i=1}^{19} |b_j - b_k|_i,$$

for all the 19 AC cases, , where j,k=1,3, and j≠k, and all the 7 AD cases,

$$\langle |b_j - b_k| \rangle_{AD} = \frac{1}{7} \sum_{i=1}^{7} |b_j - b_k|_i$$

, where j,k=1,3, and j≠k. The overall averages for the absolute differences were also cal-

$$\langle |b_j - b_k| \rangle_{AD} = \frac{1}{26} \sum_{i=1}^{26} |b_j - b_k|_i \cdots$$

culated for all 26 values, · The smaller these group differences were, the closer the two biomarkers were correlated **Fig. 8B** (Fig. 8B shows average distances between paris of biomarkers, per group).

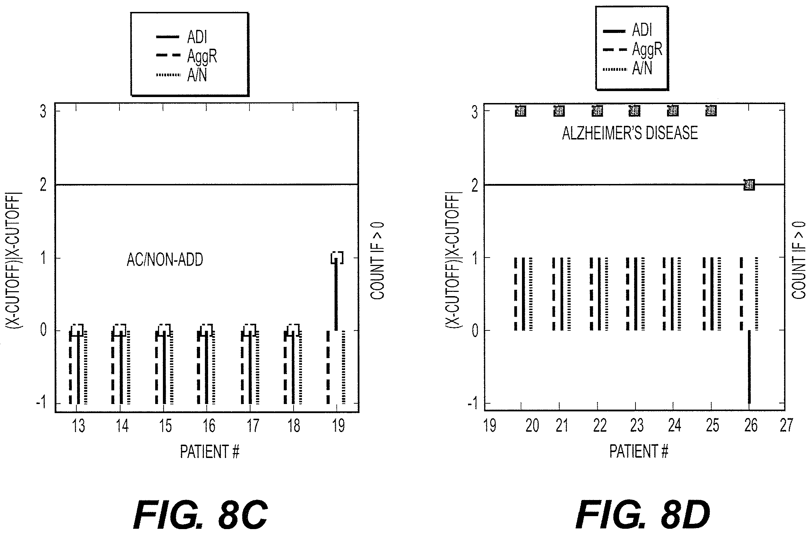

For quantifying the majority rule, once more the bio-marker values, x, were normalized with respect to the

$$\frac{(x-Cutoff)}{|x-Cutoff|}$$

specific Cutoff value, · Examples are shown in **Figs. 8C** and **8D** with the vertical bars for the 7 AC and 7 AD cases. (Fig. 8C shows 7 AC cases from **(A)** that were normalized by the cutoff values for each biomarker, where AggR=dashed lines, ADI=solid lines, and A/N=dotted lines; the empty black squares on the right y scale represent the "count if>0" for positive (+1) results of the biomarkers) and (Fig. 8D plots the same analysis as in **(C),** except for the 7 AD cases; the filled black squares on the right y scale represent the "count if>0" for the positive (+1) results of the biomarkers). There were three possible outcomes of this cutoff normalization: -1, 1, and uncertain (U). When the x value was equal with the cut-off value, an uncertain situation results, i.e. the case cannot be diagnosed and the output of the biomarker is labeled uncertain (U). This second normalization with respect to the cutoff value simplified the outcome of the biomarker into three states: +1 for AD, -1 for AC and Non-ADD, and U for cutoff values. Given three biomarkers, the possible states for the sum(S) were {3, 2, 1,-1,-2,-3, 2U, 3U} as shown in Table 1.

**Table 1: Possible states for the sum of the normalized values of the three biomarkers by the cutoff values.**

| Aggregation Rate (AggR) | AD Index (ADI) | Aggregation Area per Number (A/N) | Sum(S) | Diagnostic |
|---|---|---|---|---|
| 1 | 1 | 1 | 3 | AD |
| 1 | 1 | U* | 2 | AD |
| 1 | 1 | -1 | 1 | AD |
| 1 | -1 | -1 | -1 | AC/Non-ADD |
| -1 | -1 | U* | -2 | AC/Non-ADD |

(continued)

| Aggregation Rate (AggR) | AD Index (ADI) | Aggregation Area per Number (A/N) | Sum(S) | Diagnostic |
|---|---|---|---|---|
| -1 | -1 | -1 | -3 | AC/Non-ADD |
| +/-1 | U | U** | 2U | Not diagnosed |
| U | U | U | 3U | Not diagnosed |
| U=uncertain state which occurs when the value of the biomarker is equal with the cutoff value. *refers to the situation where one uncertain biomarker exists out of the three. **refers to the situation where two uncertain biomarkers exist out of the three. By this measure, S, there are three states: 1) AD for S=1, 2, 3; 2) AC/Non-ADD for S=-1, -2, -3; and 3) Not diagnosed for two (2U) or three (3U) uncertain, i.e., cutoff values. ||||

In **Fig. 8,** the AC cases are shown with empty symbols while the AD cases are shown with filled symbols. The AC cases showed a linear dependence for all three biomarkers, and was almost flat for aggregation rate and A/N. The AD cases showed a highly nonlinear dependence for which an exponential curve was used to fit. In **Fig. 8A,** the aggregation rate (AggR) values were ranked from minimum, 0, to maximum, 1. The AD-Index biomarker (circles) showed a significant noise especially for the AC cases.

The absolute difference between pairs of values were considered on a case by case basis to measure the closeness of the three biomarkers (**Fig. 8B**). By far the less noisy difference was |A/N-AggR|. The noise of the other two absolute differences, |AggR-ADI| and |ADI-A/N| might come from the noise in the AD-Index.

An overview of the closeness of the three biomarkers is represented in **Fig. 8B** where the average of the absolute distances is presented for the AC cases, AD cases, and overall cases. The absolute differences between the AD cases were bigger than the absolute differences between the AC cases while the overall absolute difference was in between. Again, the absolute difference between the A/N and Aggregation rate, |A/N-AggR|, was the smallest suggesting that the two biomarker measures had the strongest correlation.

The same majority-rule presented herein could be applied to more than 3 biomarkers. The three biomarker values for the 7 AC and 7 AD patients were normalized as (x-Cutoff)/|x-Cutoff| as described above, where x was the current value of the biomarker and the Cutoff was the separation between the AC and AD groups. The cutoff values were determined at the intersection of the two Gaussian distributions fitting the AC and AD data.

The normalized values as described above can have one of the three possible states -1 for AC and Non-ADD, 1 for AD, or uncertain (U) for the cutoff value. The normalized AD values for the three AD biomarkers are shown in **Fig. 8D.** For cases 19 and 26, one biomarker (AD-Index) gave a normalized value opposite to the other two biomarkers. Therefore, these cases were diagnosed correctly by following the majority rule. One way to quantify this rule is to sum up all the +1 values/per case using the simple function like count if (>0) as shown by the empty black squares in **Fig. 8C** or filled black squares in **Fig. 8D.** If the result of the "count if" is greater than or equal to 2 then the case is an AD **(Fig. 8D).** If the result of the "count if" is less than 2, then that case is an AC/Non-ADD case **(Fig. 8C).** If two or more bio-markers have the cutoff value, it cannot be diagnosed on the basis of these three biomarkers. Similarly, one can use the "count if" (<0) which is a redundant pathway. This approach is summarized in **Tables 2** and **3** below and **Figs. 8C** and **8D.**

**Table 2: Possible states for the count if (>0/<0) of the normalized values of the three biomarkers by the cutoff values**

| Aggregation Rate (AggR) | AD Index (ADI) | Aggregation Area per Number (A/N) | Count if>0 | Count if <0 (redundant) | Diagnostic |
|---|---|---|---|---|---|
| 1 | 1 | 1 | 3 | 0 | AD |
| 1 | 1 | U | 2 | 0 | AD |
| 1 | 1 | -1 | 2 | 1 | AD |
| 1 | -1 | -1 | 1 | 2 | AC/Non-ADD |
| -1 | -1 | U | 0 | 2 | AC/Non-ADD |

(continued)

| Aggregation Rate (AggR) | AD Index (ADI) | Aggregation Area per Number (A/N) | Count if>0 | Count if <0 (redundant) | Diagnostic |
|---|---|---|---|---|---|
| -1 | -1 | -1 | 0 | 3 | AC/Non-ADD |
| +/-1 | U | U | NA | NA | Not diagnosed |
| U | U | U | NA | NA | Not diagnosed |
| U=uncertain state when the value of the biomarker is equal to the cutoff value. By this measure, sum of the binary values, there are three states: 1) AD for count if (>0) = 2, 3; 2) AC/Non-ADD for count if (>0)=0, 1; and 3) Not diagnosed for two or more uncertain, i.e., cutoff values. | | | | | |

Table 3: Quantification of the majority rule for the three biomarkers normalized by the cutoff values.

| Sum of the normalized +1 values | Diagnosis |
|---|---|
| 2, 3 | Alzheimer's Disease |
| 0, 1 | Age-matched Control or Non Alzheimer's Dementia |
| 2 or more cutoff values(U) | Not diagnosed |

## Example 7: Test-Retest Reliability

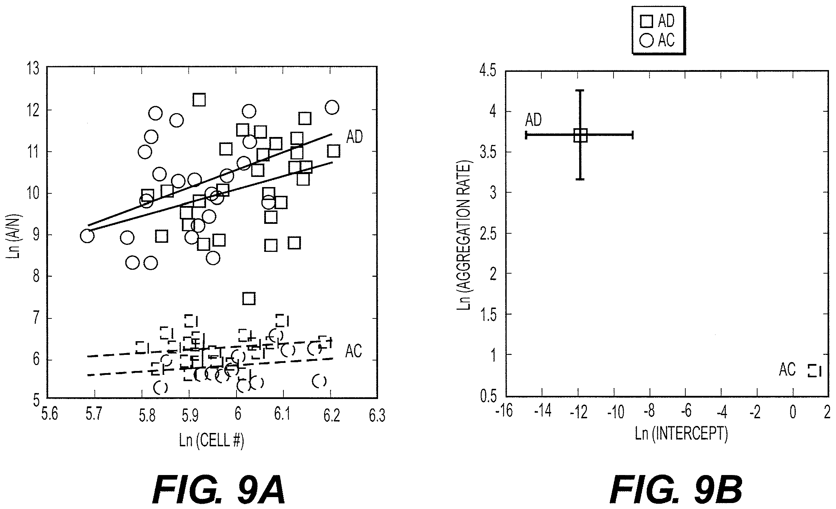

The test-retest reliability was verified for the Aggregation Rate assay with two cell lines (1 AC, 1 AD) (**Fig. 9**). First experiments (circles) were repeated for the same cell lines with a different Matrigel lot. Lines show the best fit. **Fig. 9A** plots Ln(Aggregation Rate) versus Ln(Cell#) for 1 AD case and one AC case. The average values for the Ln (Aggregation Rate) and Ln (Intercept) are presented in **Fig. 9B** (Fig. 9 Bplots Ln(Aggregation Rate) versus the Ln(Intercept) for the two cases in **(A)**), and the error-bars represent the standard error of the mean.

In summary the slope intercept representation of the human skin fibroblast aggregation showed significant separation in the slope but not in the intercept, suggesting that the elevated rate of change of cell aggregation with increasing cell density in AD was the driving force in the experiments for this new biomarker.

Analyses on 38 cases, out of which 9 were banked cases and 29 were cases from the clinic, suggested a clear separation between AD and AC/Non-ADD when using the aggregation rate (slope) as a biomarker.

The analysis of the probability distributions of the slope and intercept for 35 samples indicated that the probability distribution for the AC group (27) was narrower and resided on the tail of the probability distribution of the AD group (8) which was wider. This suggested that for larger data sets the estimated overlapping probability for the two groups, AD and AC, is less than 10%.

The new biomarker, aggregation rate (A), was cross-validated with two more mature assays, AD-Index (B), and area per number of aggregates (C). The cross-validation resulted in 92% overlap with each of the assays and in >97% overlap with the clinical diagnosis. The 92% agreement of the new biomarker A with the two more mature biomarkers B and C, which were also tested with hyper-validated samples, established this new biomarker via Euclid's common notions as a hyper-validated biomarker. See Euclid, Elements: Books I-XIII-Complete and Unabridged, (2006). That is, if biomarkers B,C = hyper-validated and if B,C = A (92%) then A= hyper-validated (92%).

Further, under a simple majority rule, i.e. when two out of the three diagnostic assays agree, the biomarkers agreed with clinical diagnosis 100%.

Supplemental Tables:

**Supplemental Table I: In-depth demographic, genetic/family history and clinical history of the Banked patients**

| | | | | Age-matched Controls (AC) (n=3) | | | |
|---|---|---|---|---|---|---|---|
| Cell ID | Demographic | Age (yrs) | Sex (M/F) | Genetic/family history | Clinical diagnosis | Biopsy source | Hyper-validated (Y/N) |
| AG11734 | Caucasian | 50 | F | NA | non-demented | mid-upper left arm | Y |
| AG07714 | Caucasian | 56 | F | NA | non-demented | mid-upper left arm | Y |
| AG05840 | Caucasian | 55 | F | NA | non-demented | mid-upper left arm | Y |

| | | | | Alzheimer's Disease (AD) (n=3) | | | |
|---|---|---|---|---|---|---|---|
| Cell ID | Demographic | Age (yrs) | Sex (M/F) | Genetic/family history | Clinical diagnosis | Biopsy source | Hyper-validated (Y/N) |
| AG06263 | Caucasian | 67 | F | history of dementia; cortical atrophy | Y | upper left arm | N |
| AG10788 | Caucasian | 87 | NA | autopsy confirmed Alzheimer's disease; homozygous for the 4 allele of apolipoprotein E | Y | right thigh | Y |
| GM00364 | Caucasian | 53 | M | family history | Y | NA | Y |

| | | | | Non-Alzheimer's Disease Dementia (Non-ADD) (n=3) | | | |
|---|---|---|---|---|---|---|---|
| Cell ID | Demographic | Age (yrs) | Sex (M/F) | Genetic/family history | Clinical diagnosis | Biopsy source | Hyper-validated (Y/N) |
| GM04715 | Caucasian | 40 | M | Huntington disease -family history | Y | NA | Y |
| GM04198 | Caucasian | 63 | F | Huntington disease - family history | Y | NA | Y |
| GM05030 | Caucasian | 56 | M | Huntington disease - choreic movemenls and dementia | Y | NA | N |

| Supplemental Table II: Information regarding the fresh clinical samples | | | | | |
|---|---|---|---|---|---|
| Age-matched Controls (AC) (n=24) | | | | | |
| Cell ID | Age(yrs) | Sex(M/F) | Clinical diagnosis | Biopsy source | Hyper-validated (Y/N) |
| 00019 | 33 | M | non-demented | under-arm | Y |
| 00025 | 39 | M | non-demented | under-arm | Y |
| 00027 | 32 | M | non-demented | under-arm | Y |

(continued)

| Supplemental Table II: Information regarding the fresh clinical samples | | | | | |
|---|---|---|---|---|---|
| Age-matched Controls (AC) (n=24) | | | | | |
| Cell ID | Age(yrs) | Sex(M/F) | Clinical diagnosis | Biopsy source | Hyper-validated (Y/N) |
| 00029 | 21 | M | non-demented | under-arm | Y |
| 00032 | 23 | M | non-demented | under-arm | Y |
| 00036 | 46 | M | non-demented | under-arm | Y |
| 00037 | 65 | F | non-demented | under-arm | Y |
| 00038 | 68 | M | non-demented | under-arm | Y |
| 00039 | 65 | F | non-demented | under-arm | Y |
| 00041 | 34 | M | non-demented | under-arm | Y |
| 00044 | 50 | F | non-demented | under-arm | Y |
| 00050 | 61 | M | non-demented | under-arm | Y |
| 00051 | 55 | M | non-demented | under-arm | Y |
| 00062 | 49 | F | non-demented | under-arm | Y |
| 00073 | 20 | F | non-demented | under-arm | Y |
| 00075 | 38 | F | non-demented | under-arm | Y |
| 00076 | 36 | M | non-demented | under-arm | Y |
| 00077 | 18 | M | non-demented | under-arm | Y |
| 00078 | 45 | F | non-demented | under-arm | Y |
| 00079 | 25 | M | non-demented | under-arm | Y |
| 00080 | 45 | M | non-demented | under-arm | Y |
| 00081 | 42 | F | non-demented | under-arm | Y |
| 00082 | 45 | F | non-demented | under-arm | Y |
| 00084 | 21 | M | non-demented | under-arm | Y |
| | | | | | |
| Alzheimer's Disease (AD) (n=5) | | | | | |
| Celll ID | Age(yrs) | Sex(M/F) | Clinical diagnosis | Biopsy source | Hyper-validated (Y/N) |
| 00040 | 59 | M | Y | under-arm | N |
| 00042 | 78 | M | Y | under-arm | N |
| 00043 | 88 | M | Y | under-arm | N |
| 00060 | 81 | M | Y | under-arm | N |
| 00061 | 79 | M | Y | under-arm | N |

## Claims

1. A method of diagnosing Alzheimer's Disease in a human subject comprising the steps of

   (a) culturing one or more cells that had been obtained from a human subject for a time period;
   (b) determining an average area of cell aggregates and dividing the average area by the number of aggregates to obtain an average area per number of aggregates;
   (c) determining an aggregation rate by evaluating the rate of change of the average area per number of aggregates determined in step (b) as a function of cell density, wherein the rate of change of the average per number of aggregates as a function of cell density is evaluated by determining the slope of a linear fit between the average area per number of aggregates and cell density;

(d) comparing the determination of step (c) with an aggregation rate determined using non-Alzheimer's disease cells; and

(e) diagnosing the presence or absence of Alzheimer's Disease based on the comparison in step (d), wherein the diagnosis is positive for Alzheimer's disease if the aggregation rate determined in step (c) is increased compared to the aggregation rate determined using the non-Alzheimer's disease cells.

2. The method of claim 1, wherein the diagnosis is confirmed using at least one additional diagnostic assay, optionally wherein the at least one additional diagnostic assay is chosen from AD-Index and Morphology diagnostic assays.

3. The method of claim 1, wherein the non-Alzheimer's disease cells are chosen from an aged-matched control.

4. The method of claim 1, wherein the time period is chosen from about 12 hours to about 72 hours, optionally from about 24 hours to about 48 hours, optionally wherein the time period is about 48 hours.

5. The method of claim 1, further comprising imaging the cultured cells at the end of the time period.

6. The method of claim 5, wherein the cell density is measured by measuring the number of cells per 10× image.

7. The method of claim 5, wherein the rate of change of the average area per number of aggregates as a function of cell density is evaluated within the boundaries of 320 to 550 cells/10× image.

8. The method of claim 1, wherein the one or more cells are fibroblast cells, optionally skin fibroblast cells.

9. The method of claim 1, wherein the one or more cells are cultured in a preparation comprising extracellular matrix proteins.

10. The method of claim 9, wherein the preparation comprises laminin, collagen, heparin sulfate proteoglycans, entactin/nidogen, and/or combinations thereof, or a growth factor.

11. The method of claim 9, wherein the preparation is extracted from a tumor, optionally wherein the tumor is the EHS mouse sarcoma.

12. A method of diagnosing Alzheimer's disease in a human subject comprising

i) performing at least three diagnostic assays;
ii) evaluating whether each assay indicates the presence or absence of Alzheimer's disease; and
iii) diagnosing the presence or absence of Alzheimer's disease based on whether a simple majority of the diagnostic assays indicate the presence or absence of Alzheimer's disease,

wherein one of the diagnostic assays comprises:

(a) culturing one or more cells that had been obtained from a human subject for a time period;
(b) determining an average area of cell aggregates and dividing the average area by the number of aggregates to obtain an average area per number of aggregates;
(c) determining an aggregation rate by evaluating the rate of change of the average area per number of aggregates determined in step (b) as a function of cell density, wherein the rate of change of the average per number of aggregates as a function of cell density is evaluated by determining the slope of a linear fit between the average area per number of aggregates and cell density; and
(d) comparing the determination of step (c) with an aggregation rate determined using non-Alzheimer's Disease cells, wherein the presence of Alzheimer's disease is indicated if the aggregation rate determined in step (c) is increased compared to the aggregation rate determined using the non-Alzheimer's Disease cells.

13. The method of claim 12, wherein two of the at least three diagnostic assays are AD-Index and Morphology assays.

14. The method of claim 12, wherein the one or more cells are fibroblast cells, optionally skin fibroblast cells.

**EP 3 090 261 B1**

**Patentansprüche**

1. Verfahren zum Diagnostizieren von Alzheimer in einem menschlichen Subjekt, die folgenden Schritte umfassend:

   (a) Kultivieren einer oder mehrerer Zellen, die von einem menschlichen Subjekt über einen Zeitraum erhalten wurden;
   (b) Bestimmen eines durchschnittlichen Bereichs von Zellaggregaten und Teilen des durchschnittlichen Bereichs durch die Anzahl von Aggregaten, um einen durchschnittlichen Bereich pro Anzahl von Aggregaten zu erhalten;
   (c) Bestimmen einer Aggregationsgeschwindigkeit durch Beurteilen der Änderungsgeschwindigkeit des in Schritt (b) bestimmten durchschnittlichen Bereichs pro Anzahl von Aggregaten als eine Zelldichtefunktion, wobei die Änderungsgeschwindigkeit der durchschnittlichen Anzahl von Aggregaten als eine Zelldichtefunktion durch das Bestimmen des Anstiegs einer linearen Anpassung zwischen dem durchschnittlichen Bereich pro Anzahl von Aggregaten und der Zelldichte beurteilt wird;
   (d) Vergleichen der Bestimmung von Schritt (c) mit einer Aggregationsgeschwindigkeit, die unter Verwendung von Zellen bestimmt wird, die nicht an Alzheimer erkrankt sind; und
   (e) Diagnostizieren der Gegenwart oder der Abwesenheit von Alzheimer basierend auf dem Vergleich in Schritt (d), wobei die Diagnose für Alzheimer positiv ist, falls die in Schritt (c) bestimmte Aggregationsgeschwindigkeit im Vergleich zu der Aggregationsgeschwindigkeit, die unter Verwendung der Zellen bestimmt wird, die nicht an Alzheimer erkrankt sind, erhöht ist.

2. Verfahren nach Anspruch 1, wobei die Diagnose unter Verwendung mindestens eines zusätzlichen diagnostischen Tests bestätigt wird, wobei der mindestens eine zusätzliche diagnostische Test gegebenenfalls aus AD-Index-Diagnosetests und morphologischen Diagnosetests ausgewählt ist.

3. Verfahren nach Anspruch 1, wobei die Zellen, die nicht an Alzheimer erkrankt sind, aus einer gleichaltrigen Kontrolle ausgewählt sind.

4. Verfahren nach Anspruch 1, wobei der Zeitraum von etwa 12 Stunden bis etwa 72 Stunden ausgewählt ist, gegebenenfalls von etwa 24 Stunden bis etwa 48 Stunden, wobei der Zeitraum gegebenenfalls 48 Stunden beträgt.

5. Verfahren nach Anspruch 1, ferner das Abbilden der kultivierten Zellen am Ende des Zeitraums umfassend.

6. Verfahren nach Anspruch 5, wobei die Zelldichte durch das Messen der Anzahl von Zellen pro 10x-Abbildung gemessen wird.

7. Verfahren nach Anspruch 5, wobei die Änderungsgeschwindigkeit des durchschnittlichen Bereichs pro Anzahl von Aggregaten als eine Zelldichtefunktion innerhalb der Grenzen von 320 bis 550 Zelle/10x-Abbildung beurteilt wird.

8. Verfahren nach Anspruch 1, wobei die eine oder mehreren Zellen Fibroblastzellen, gegebenenfalls Hautfibroblastzellen, sind.

9. Verfahren nach Anspruch 1, wobei die eine oder mehreren Zellen in einem Präparat kultiviert werden, das extrazelluläre Matrixproteine umfasst.

10. Verfahren nach Anspruch 9, wobei das Präparat Laminin, Kollagen, Heparinsulfatproteoglykane, Entactin/Nidogen und/oder Kombinationen davon oder einen Wachstumsfaktor umfasst.

11. Verfahren nach Anspruch 9, wobei das Präparat aus einem Tumor extrahiert ist, wobei der Tumor gegebenenfalls das EHS-Maussarkom ist.

12. Verfahren zum Diagnostizieren von Alzheimer in einem menschlichen Subjekt, Folgendes umfassend:

    i) Durchführen von mindestens drei diagnostischen Tests;
    ii) Beurteilen, ob jeder Test die Gegenwart oder die Abwesenheit von Alzheimer anzeigt; und
    iii) Diagnostizieren der Gegenwart oder der Abwesenheit von Alzheimer basierend darauf, ob eine einfache Mehrheit der diagnostischen Tests die Gegenwart oder die Abwesenheit von Alzheimer anzeigt, wobei einer der diagnostischen Tests Folgendes umfasst:

(a) Kultivieren einer oder mehrerer Zellen, die von einem menschlichen Subjekt über einen Zeitraum erhalten wurden;

(b) Bestimmen eines durchschnittlichen Bereichs von Zellaggregaten und Teilen des durchschnittlichen Bereichs durch die Anzahl von Aggregaten, um einen durchschnittlichen Bereich pro Anzahl von Aggregaten zu erhalten;

(c) Bestimmen einer Aggregationsgeschwindigkeit durch Beurteilen der Änderungsgeschwindigkeit des in Schritt (b) bestimmten durchschnittlichen Bereichs pro Anzahl von Aggregaten als eine Zelldichtefunktion, wobei die Änderungsgeschwindigkeit der durchschnittlichen Anzahl von Aggregaten als eine Zelldichtefunktion durch das Bestimmen des Anstiegs einer linearen Anpassung zwischen dem durchschnittlichen Bereich pro Anzahl von Aggregaten und der Zelldichte beurteilt wird; und

(d) Vergleichen der Bestimmung von Schritt (c) mit einer Aggregationsgeschwindigkeit, die unter Verwendung von Zellen bestimmt wird, die nicht an Alzheimer erkrankt sind, wobei die Gegenwart von Alzheimer angezeigt wird, falls die in Schritt (c) bestimmte Aggregationsgeschwindigkeit im Vergleich mit der Aggregationsgeschwindigkeit, die unter Verwendung der Zellen bestimmt wird, die nicht an Alzheimer erkrankt sind, erhöht ist.

**13.** Verfahren nach Anspruch 12, wobei zwei der mindestens drei diagnostischen Tests AD-Index-Tests und morphologische Tests sind.

**14.** Verfahren nach Anspruch 12, wobei die eine oder mehreren Zellen Fibroblastzellen, gegebenenfalls Hautfibroblastzellen, sind.


**Revendications**

**1.** Procédé de diagnostic de la maladie d'Alzheimer chez un sujet humain comprenant les étapes de

(a) culture d'une ou de plusieurs cellules qui ont été obtenues à partir d'un sujet humain pendant une période de temps ;

(b) détermination d'une superficie moyenne d'agrégats de cellules et division de la superficie moyenne par le nombre d'agrégats pour obtenir une superficie moyenne par nombre d'agrégats ;

(c) détermination d'un taux d'agrégation en évaluant le taux de changement de la superficie moyenne par nombre d'agrégats déterminée dans l'étape (b) comme une fonction de la densité cellulaire, dans lequel le taux de changement de la moyenne par nombre d'agrégats comme une fonction de la densité cellulaire est évalué en déterminant la pente d'un ajustement linéaire entre la superficie moyenne par nombre d'agrégats et la densité cellulaire ;

(d) comparaison de la détermination de l'étape (c) avec un taux d'agrégation déterminé en utilisant des cellules qui ne sont pas de la maladie d'Alzheimer ; et

(e) diagnostic de la présence ou de l'absence de la maladie d'Alzheimer sur la base de la comparaison dans l'étape (d), dans lequel le diagnostic est positif pour la maladie d'Alzheimer si le taux d'agrégation déterminé dans l'étape (c) est augmenté par rapport au taux d'agrégation déterminé en utilisant les cellules qui ne sont pas de la maladie d'Alzheimer.

**2.** Procédé selon la revendication 1, dans lequel le diagnostic est confirmé en utilisant au moins un dosage de diagnostic supplémentaire, facultativement dans lequel l'au moins un dosage de diagnostic supplémentaire est choisi parmi les dosages de l'index MA et de diagnostic morphologique.

**3.** Procédé selon la revendication 1, dans lequel les cellules qui ne sont pas de la maladie d'Alzheimer sont choisies à partir d'un témoin d'un âge correspondant.

**4.** Procédé selon la revendication 1, dans lequel la période de temps est choisie parmi environ 12 heures à environ 72 heures, facultativement environ 24 heures à environ 48 heures, facultativement dans lequel la période de temps est d'environ 48 heures.

**5.** Procédé selon la revendication 1, comprenant en outre l'imagerie des cellules cultivées à la fin de la période de temps.

**6.** Procédé selon la revendication 5, dans lequel la densité cellulaire est mesurée en mesurant le nombre de cellules sur image 10 x.

**7.** Procédé selon la revendication 5, dans lequel le taux de changement de la superficie moyenne par nombre d'agrégats comme une fonction de la densité cellulaire est évalué dans les limites de 320 à 550 cellules / image 10 x.

**8.** Procédé selon la revendication 1, dans lequel les une ou plusieurs cellules sont des fibroblastes, facultativement des fibroblastes cutanés.

**9.** Procédé selon la revendication 1, dans lequel les une ou plusieurs cellules sont cultivées dans une préparation comprenant des protéines matricielles extracellulaires.

**10.** Procédé selon la revendication 9, dans lequel la préparation comprend de la laminine, du collagène, des protéoglycanes à sulfate d'héparine, de l'entactine/nidogène, et/ou des combinaisons de ceux-ci, ou un facteur de croissance.

**11.** Procédé selon la revendication 9, dans lequel la préparation est extraite d'une tumeur, facultativement dans lequel la tumeur est un sarcome murin EHS.

**12.** Procédé de diagnostic de la maladie d'Alzheimer chez un sujet humain comprenant :

i) la réalisation d'au moins trois dosages de diagnostic ;
ii) le fait d'évaluer si chaque dosage indique la présence ou l'absence de la maladie d'Alzheimer ; et
iii) le diagnostic de la présence ou de l'absence de la maladie d'Alzheimer sur la base du fait qu'une simple majorité des dosages de diagnostic indiquent la présence ou l'absence de la maladie d'Alzheimer, dans lequel un des dosages de diagnostic comprend :

(a) la culture d'une ou de plusieurs cellules qui ont été obtenues à partir d'un sujet humain pendant une période de temps ;
(b) la détermination d'une superficie moyenne d'agrégats de cellules et la division de la superficie moyenne par le nombre d'agrégats pour obtenir une superficie moyenne par nombre d'agrégats ;
(c) la détermination d'un taux d'agrégation en évaluant le taux de changement de la superficie moyenne par nombre d'agrégats déterminée dans l'étape (b) comme une fonction de la densité cellulaire, dans lequel le taux de changement de la moyenne par nombre d'agrégats comme une fonction de la densité cellulaire est évalué en déterminant la pente d'un ajustement linéaire entre la superficie moyenne par nombre d'agrégats et la densité cellulaire ; et
(d) la comparaison de la détermination de l'étape (c) avec un taux d'agrégation déterminé en utilisant des cellules qui ne sont pas de la maladie d'Alzheimer, dans lequel la présence de la maladie d'Alzheimer est indiquée si le taux d'agrégation déterminé dans l'étape (c) est augmenté par rapport au taux d'agrégation déterminé en utilisant les cellules qui ne sont pas de la maladie d'Alzheimer.

**13.** Procédé selon la revendication 12, dans lequel deux des au moins trois dosages de diagnostic sont des dosages de l'index MA et de morphologie.

**14.** Procédé selon la revendication 12, dans lequel les une ou plusieurs cellules sont des fibroblastes, facultativement des fibroblastes cutanés.

EP 3 090 261 B1

**FIG. 1A**

**FIG. 1B**

**FIG. 1C**

**FIG. 1D**

*FIG. 1F*

*FIG. 1E*

FIG. 2B

FIG. 2A

FIG. 2D

FIG. 2C

*FIG. 3A*

*FIG. 3B*

*FIG. 3D*

*FIG. 3C*

FIG. 3E

FIG. 3F

FIG. 4B

FIG. 4A

**FIG. 4D**

**FIG. 4C**

FIG. 5B

FIG. 5A

FIG. 6A

FIG. 6B

**FIG. 6C**

**FIG. 6D**

% CORRECT CASES

100

92

AGGR. RATE
AND AD INDEX

AGGR. RATE AND
CLINICAL D.

**FIG. 6E**

% CORRECT CASES

92

97

AGGR. RATE
AND A/N

AGGR. RATE AND
CLINICAL D.

**FIG. 6F**

EP 3 090 261 B1

**FIG. 7A**

**FIG. 7B**

*FIG. 8B*

*FIG. 8A*

FIG. 8D

FIG. 8C

35

FIG. 9B

FIG. 9A

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61923373 A
- WO 2012155051 A
- US 7595167 B
- US 20140031245 A
- US 8658134 B

**Non-patent literature cited in the description**

- **WHALLEY K.** Neurodegenerative disease: Dishing up Alzheimer's disease. *Nature Reviews Neuroscience,* March 2012, vol. 13, 149
- **CHIRILA F.V. et al.** Spatiotemporal Complexity of Fibroblast Networks Screens for Alzheimer's Disease. *J. Alzheimer's Disease,* 2013, vol. 33, 165-176
- **ZHAO W. Q. et al.** MAP kinase signaling cascade dysfunction specific to Alzheimer's disease in fibroblasts. *Neurobiol. Dis.,* 2002, vol. 11, 166-183
- **KHAN T. K. et al.** An internally controlled peripheral biomarker for Alzheimer's disease: Erk1 and Erk2 responses to the inflammatory signal bradykinin. *Proc. Natl. Acad. Sci. U.S.A.,* 2006, vol. 103 (35), 13203-7
- **KHAN T. K. et al.** Early diagnostic accuracy and pathophysiologic relevance of an autopsy-confirmed Alzheimer's disease peripheral biomarker. *Neurobiol. Aging,* 2008, vol. 31 (6), 889-900
- **KHAN T. K. et al.** A cellular model of Alzheimer's disease therapeutic efficacy: PKC activation revesres Abeta-induced biomarker abnormality on cultured fibroblasts. *Neurobiol. Dis.,* 2009, vol. 34 (2), 332-9
- **CHIRILA F. V. et al.** Spatiotemporal Complexity of Fibroblast Networks Screens for Alzheimer's Disease. *J. Alzheimer's Disease,* 2013, vol. 33, 165-176
- **TAKASHIMA, A.** Establishment of Fibroblast Cultures. *Current Protocols in Cell Biology,* 1998, vol. 2, 2.1.1-2.1.12
- **BIKFALVI A. et al.** Phenotypic modulations of human umbilical vein endothelial cells and human dermal fibroblasts using two angiogenic assays. *Biol. Cell,* 1991, vol. 72, 275-278
- **HADLEY MA et al.** Extracellular matrix regulates Sertoli cell differentiation, testicular cord formation and germ cell development. *J. Cell Biol.,* 1985, vol. 101, 1511-1522
- **INGBER D. et al.** Mechanochemical switching between growth and differentiation during fibroblast growth factor-stimulated angiogenesis in vitro. Role of extracellular matrix. *J. Cell Biol.,* 1989, vol. 109, 317-331
- **FURUKAWA KS et al.** Tissue-engineered skin using aggregates of normal human skin fibroblasts and biodegradable material. *J. Artif. Organs,* 2001, vol. 4, 353-356
- **FURUKAWA KS et al.** Formation of human fibroblast aggregates (spheroids) by rotational culture. *Cell Transplantation,* 2001, vol. 10, 441-445
- **EUCLID.** Elements: Books I-XIII-Complete and Unabridged. 2006